# EUROPÄISCHE PATENTSCHRIFT

(11) **EP 3 038 632 B1**
(45) Veröffentlichungstag und Bekanntmachung des Hinweises auf die Patenterteilung: **27.09.2017**
(21) Anmeldenummer: 15700546.3
(22) Anmeldetag: 12.01.2015
(51) Int. Cl.: A61K 36/16

(54) **VERBESSERTES VERFAHREN ZUR HERSTELLUNG VON GINKGOEXTRAKTEN**
IMPROVED METHOD FOR PRODUCING GINKGO EXTRACTS
PROCÉDÉ AMÉLIORÉ POUR LA PRODUCTION D'EXTRAITS DE GINKGO

(30) Priorität: 10.02.2014 DE 102014202318
(43) Veröffentlichungstag der Anmeldung: 06.07.2016
(73) Patentinhaber: Dr. Willmar Schwabe GmbH & Co. KG, 76227 Karlsruhe (DE)
(72) Erfinder: WAIMER, Frank, 76228 Karlsruhe (DE); REINHARD, Steffen, 76227 Karlsruhe (DE); HAUER, Hermann, 76228 Karlsruhe (DE)
(74) Vertreter: Adam, Holger
(86) Internationale Anmeldenummer: PCT/EP2015/050401
(87) Internationale Veröffentlichungsnummer: WO 2015/117793

(56) Entgegenhaltungen:
- EP-A1- 1 868 568
- EP-A1- 1 868 625
- WO-A1-2012/146592
- DE-A1- 3 940 092
- DENG F ET AL: "Development and validation of a gas chromatographic-mass spectrometric method for simultaneous identification and quantification of marker compounds including bilobalide, ginkgolides and flavonoids in Ginkgo biloba L. extract and pharmaceutical preparations", JOURNAL OF CHROMATOGRAPHY A 20030131 NL, Bd. 986, Nr. 1, 31. Januar 2003 (2003-01-31), Seiten 121-127, XP4401771, ISSN: 0021-9673

## Beschreibung

Die vorliegende Erfindung betrifft ein verbessertes vielstufiges Verfahren zur Herstellung eines Extrakts aus Blättern von Ginkgo biloba zur Verwendung als Arzneimittel.

Extrakte aus den Blättern von Ginkgo biloba werden seit Jahrzehnten als Arzneimittel verwendet. Derzeit werden sie zur Behandlung verschiedener Arten von Demenz und deren Symptome sowie von cerebralen und peripheren Durchblutungsstörungen verwendet. Inhaltsstoffe, mit denen die Wirksamkeit verknüpft ist, sind Terpenlaktone (Ginkgolide A, B, C und Bilobalid) sowie Glycoside von Flavonen (Quercetin, Kämpferol und Isorhamnetin). Gemäß dem aktuellen Europäischen Arzneibuch (Version 8.0; Monographie 04/2008: 1827 "Ginkgo dry extract, refined and quantified"), welches verbindlich ist für alle Ginkgoextrakte, die im Geltungsbereich des Arzneibuchs als Arzneimittel zugelassen werden können, enthält Ginkgoextrakt 22.0 % bis 27.0 % Flavonoide, berechnet als Flavonglycoside, 2.6 % bis 3.2 % Bilobalid, 2.8 % bis 3.4 % Ginkgolide A, B und C sowie höchstens 5 ppm Ginkgolsäuren. Die Verstellung wird in der "Bekanntmachung über die Zulassung und Registrierung von Arzneimittel (Aufbereitungsmonographien für den humanmedizinischen Bereich) - Ginkgo folium (Ginkgo-biloba-Blätter)" (Bundesanzeiger 46, (133), 7360-7361 (1994)) folgendermaßen beschrieben: "Ein aus den getrockneten Blättern von Ginkgo biloba LINNE mit Aceton-Wasser und nachfolgenden Reinigungsschritten ohne Zumischung von Konzentraten oder isolierten Inhaltsstoffen hergestellter Trockenextrakt".

Man erhält in letzter Zeit vermehrt Ginkgoblätter mit Gehalten an Inhaltsstoffen, die bei der Extraktion mit den bekannten Verfahren nicht zu Extrakten führen, die den Anforderungen des Europäischen Arzneibuchs entsprechen. Insbesondere werden derzeit vermehrt Ginkgoblätter mit ungewöhnlich hohen Gehalten an Terpenlaktonen geerntet, die entsprechend erhöhte Gehalte im Extrakt zur Folge haben. Gelegentlich enthalten Ginkgoblätter jedoch auch eine ungewöhnlich hohe Menge an Flavonoiden.

Die wichtigsten Extraktionsverfahren, die bei Einsatz geeigneter Ginkgo-Blätter zu Extrakten gemäß den Vorgaben des Ph. Eur. führen, sind beschrieben in der EP 431535 B1 (Dr. Willmar Schwabe GmbH & Co.) und in der EP 360556 B1 (Indena S.p.A.). Zunehmend an Bedeutung hat in der jüngeren Vergangenheit das Verfahren gemäß EP 431536 B1 (Dr. Willmar Schwabe GmbH & Co.) gewonnen, da bei diesem Verfahren keine toxikologisch bedenklichen Stoffe wie Bleisalze (EP 431535 B1) oder aromatische Kohlenwasserstoffe (EP 360556 B1) benötigt werden.

Gemäß Anspruch 1 der EP 431536 B1 / DE 3940092 A1 ist das darin beanspruchte Verfahren zur Herstellung eines Extraktes aus Ginkgo biloba-Blättern dadurch gekennzeichnet, dass man
(a) frische oder getrocknete grüne Blätter von Ginkgo biloba mit wasserhaltigem Aceton, einem wasserhaltigem Alkanol mit 1 bis 3 C-Atomen oder wasserfreiem Methanol bei einer Temperatur von etwa 40 bis 100 °C extrahiert,
(b) aus dem Extrakt die Hauptmenge des organischen Lösungsmittels auf einen Gehalt von höchstens 10 Gew.-% abtrennt, wobei bei den letzten Destillationsstufen gegebenenfalls Wasser zugesetzt wird,
(c) die verbleibende konzentrierte wässrige Lösung mit Wasser auf einen Feststoffgehalt von 5 bis 25 Gew.-% verdünnt, unter Rühren auf eine Temperatur unter 25 °C abkühlt, bis zur Bildung eines Niederschlages stehen läßt und den entstandenen Niederschlag, bestehend aus den in Wasser schwer löslichen lipophilen Bestandteilen, abtrennt,
(d) die verbleibende wässrige Lösung mit Ammoniumsulfat versetzt und die entstandene Lösung mit Methylethylketon oder einem Gemisch aus Methylethylketon und Aceton extrahiert,
(e) den erhaltenen Extrakt auf einen Feststoffgehalt von 50 bis 70 % konzentriert und das erhaltene Konzentrat mit Wasser auf einen Feststoffgehalt von 5 bis 20 % verdünnt,
(f) die so erhaltene Lösung mehrstufig mit einem mit Wasser nicht mischbaren Butanol oder Pentanol extrahiert,
(g) die Butanol- bzw. Pentanolphasen auf einen Feststoffgehalt von 50 bis 70 % konzentriert,
(h) das Konzentrat durch Zugabe derartiger Mengen von Wasser und Ethanol verdünnt, dass eine Lösung von 5 bis 20 Gew.-% Trockenextrakt in 20 bis 60 Gew.-% wässrigem Ethanol erhalten wird,
(i) die wässrig-alkoholische Lösung mit einem aliphatischen oder cycloaliphatischen Lösungsmittel mit einem Siedepunkt von 60 bis 100 °C zur weiteren Abtrennung der Alkylphenol-Verbindungen extrahiert,
(j) die Wasserphase unter vermindertem Druck konzentriert und bei einer Temperatur von höchstens 60 bis 80 °C zu einem Trockenextrakt mit einem Wassergehalt von weniger als 5 % trocknet.

Aufgabe der vorliegenden Erfindung ist es, ein verbessertes Verfahren zur Herstellung eines Extraktes aus Blättern von Ginkgo biloba bereitzustellen, das die Vorgaben des Ph. Eur. auch dann erfüllt, wenn Ginkgo-Blätter mit ungünstigem Inhaltsstoffspektrum, insbesondere mit erhöhten Gehalten an Terpenlaktonen, aber auch mit erhöhtem Gehalt an Flavonoiden, zum Einsatz kommen. Darüber hinaus ist es Aufgabe der vorliegenden Erfindung, das Verfahren so zu führen, dass keine Reinstoffe oder reinstoffnahe Konzentrate zugesetzt werden, weil dies bei arzneilich genutzten Ginkgoextrakten nicht zulässig ist.

Überraschenderweise wurde nun festgestellt, dass durch zusätzliche Verfahrensschritte in dem Verfahren gemäß EP 431 536 B1 auch aus Ginkgo-Blättern mit ungünstigem Inhaltsstoffspektrum Extrakte erhalten werden, die dem Europäischen Arzneibuch entsprechen. Hierzu wird nach der Extraktion (Flüssig-Flüssig-Verteilung) gemäß Schritt (d) die wässrige Phase von der Methylethylketon- bzw. Methylethylketon-Aceton-Phase getrennt, die Methylethylketon- bzw. Methylethylketon-Aceton-Phase bis zu einem Trockenextraktanteil von 40 bis 80 Gew.-% eingeengt, um ein Konzentrat zu erhalten. Ein Anteil von 10 bis 60 Gew.-% des Konzentrats wird mit Wasser und Methylethylketon auf einen Trockenextraktanteil von höchstens 60 Gew.-% und einen Methylethylketongehalt von höchstens 30 Gew.-% eingestellt und mit einem Gemisch aus Methylethylketon und einem aliphatischen Lösungsmittel mit einem Siedepunkt von 60 bis 100°C im Verhältnis 7/3 bis 9/1 (m/m) extrahiert, um eine Wasser-Methylethylketon-Phase und eine Methylethylketon-aliphatisches Lösungsmittel-Phase zu erhalten.

Die Wasser-Methylethylketon-Phase wird mit den verbliebenen 90 bis 40 Gew.-% Konzentrat vereinigt, um eine Lösung zu erhalten, und so einem zu hohen Gehalt an Terpenlaktonen im Endprodukt entgegenzuwirken.

Alternativ kann die erhaltene Methylethylketon-aliphatisches Lösungsmittel-Phase mit den verbliebenen 90 bis 40 Gew.-% Konzentrat vereinigt werden, um einem zu hohen Gehalt an Flavonoiden im Endprodukt entgegenzuwirken.

Das erfindungsgemäße Verfahren zur Herstellung eines Trockenextraktes aus Ginkgo biloba-Blättern mit einem Gehalt an 22,0 Gew.-% bis 27,0 Gew.-% Flavonoiden, berechnet als Flavonglycoside, 2,6 Gew.-% bis 3,2 Gew.-% Bilobalid, 2,8 Gew.-% bis 3,4 Gew.-% Ginkgolide A, B und C sowie höchstens 5 ppm Ginkgolsäuren umfasst demnach folgende Schritte:
(a) Extraktion frischer oder getrockneter grüner Blätter von Ginkgo biloba mit wasserhaltigem Aceton, einem wasserhaltigen Alkanol mit 1 bis 3 C-Atomen oder wasserfreiem Methanol bei einer Temperatur von etwa 40 bis 100°C, um eine Rohextraktlösung zu erhalten,
(b) Abtrennen durch Destillation der Hauptmenge des Acetons oder Alkanols mit 1 bis 3 C-Atomen aus der Rohextraktlösung aus Schritt (a) auf einen Gehalt von höchstens 10 Gew.-%, wobei im Fall der Verwendung von wasserfreiem Methanol im Schritt (a), bei den letzten Destillationsstufen Wasser zugesetzt wird, um eine konzentrierte wässrige Lösung zu erhalten,
(c) Verdünnen der konzentrierten wässrigen Lösung aus Schritt (b) mit Wasser auf einen Feststoffgehalt von 5 bis 25 Gew.-%, Abkühlen auf eine Temperatur unter 25°C, Kühlhalten bis zur Bildung eines Niederschlags und Abtrennen des entstandenen Niederschlags, um wiederum eine wässrige Lösung zu erhalten,
(d) Versetzen der erhaltenen wässrigen Lösung aus Schritt (c) mit Ammoniumsulfat und Extraktion der entstandenen Ammoniumsulfat-enthaltenden Lösung mit Methylethylketon oder einem Gemisch aus Methylethylketon und Aceton, Trennung der wässrigen Phase von der Methylethylketon- oder Methylethylketon-Aceton-Phase, um eine Methylethylketon- oder Methylethylketon-Aceton-Phase zu erhalten,
(e) Einengen der Methylethylketon- oder Methylethylketon-Aceton-Phase aus Schritt (d) bis zu einem Trockenextraktanteil von 40 bis 80 Gew.-%, um ein Konzentrat zu erhalten,
(f) Einstellen eines Anteils von 10 bis 60 Gew.-% des Konzentrats aus Schritt (e) mit Wasser und Methylethylketon auf einen Trockenextraktanteil von höchstens 60 Gew.-% und einen Methylethylketongehalt von höchstens 30 Gew.-%, um eine eingestellte Extraktionslösung zu erhalten, und Extraktion der erhaltenen eingestellten Extraktionslösung mit einem Gemisch aus Methylethylketon und einem aliphatischen Lösungsmittel mit einem Siedepunkt von 60 bis 100°C im Verhältnis 7/3 bis 9/1 (m/m), um eine Wasser-Methylethylketon-Phase und eine Methylethylketon-aliphatisches Lösungsmittel-Phase zu erhalten,
(g) Vereinigen des verbliebenen Anteils von 90 bis 40 Gew.-% des Konzentrats aus Schritt (e) mit der Wasser-Methylethylketon-Phase aus Schritt (f) oder der Methylethylketon-aliphatisches Lösungsmittel-Phase aus Schritt (f), um eine Lösung zu erhalten,
(h) Konzentration der Lösung aus Schritt (g) auf einen Feststoffgehalt von 50 bis 70 Gew.-% und Verdünnen des erhaltenen Konzentrats mit Wasser auf einen Feststoffgehalt von höchstens 50 Gew.-%, um eine Lösung zu erhalten,
(i) mehrstufige Extraktion der in Schritt (h) erhaltenen Lösung mit einem mit Wasser nicht mischbaren Butanol oder Pentanol, um eine Butanol- oder Pentanolphase zu erhalten,
(j) Konzentration der Butanol- oder Pentanolphase aus Schritt (i) auf einen Feststoffgehalt von mindestens 50 Gew.-%, um ein Konzentrat zu erhalten,
(k) Verdünnen des in Schritt (j) erhaltenen Konzentrats durch Zugabe derartiger Mengen von Wasser und gegebenenfalls Ethanol, dass eine Lösung von 5 bis 20 Gew.-% Trockenextrakt in Wasser oder höchstens 60 Gew.-% wässrigem Ethanol erhalten wird,
(l) Extraktion der wässrigen oder wässrig-ethanolischen Lösung aus Schritt (k) mit einem aliphatischen Lösungsmittel mit einem Siedepunkt von 60 bis 100°C, Trennung der wässrigen Phase von der aliphatischenLösungsmittel-Phase, um eine Wasserphase zu erhalten,
(m) Konzentration der in Schritt (I) erhaltenen Wasserphase unter vermindertem Druck und bei einer Temperatur von höchstens 60 bis 80°C, um einen Trockenextrakt mit einem Wassergehalt von weniger als 5 Gew.-% zu erhalten. In bevorzugten Ausführungsformen der Erfindung wird
   - als Extraktionslösungsmittel in Schritt (a) wasserhaltiges Aceton mit einem Acetongehalt von etwa 50 bis 70 Gew.-%, besonders bevorzugt mit einem Acetongehalt von etwa 60 Gew.-% eingesetzt,
   - als Extraktionslösungsmittel in Schritt (a) ein wasserhaltiges Alkanol ausgewählt aus Methanol, Ethanol, 1-Propanol und 2-Propanol mit einem Alkanolgehalt von etwa 50 Gew.-% bis 70 Gew.-%, besonders bevorzugt wasserhaltiges Ethanol mit einem Ethanolgehalt von etwa 60 Gew.-% eingesetzt,
   - in Schritt (c) die verdünnte wässrige Lösung auf eine Temperatur unter 12 °C abgekühlt,
   - in Schritt (d) mindestens 30 Gew.-% Ammoniumsulfat, besonders bevorzugt 30 bis 50 Gew.-% Ammoniumsulfat bezogen auf die wässrige Lösung aus Schritt (c) zugesetzt,
   - in Schritt (d) mit einem Gemisch aus Methylethylketon und Aceton im Verhältnis von 7/3 bis 3/4 (m/m) extrahiert,
   - in Schritt (i) mit 1-Butanol extrahiert und/oder
   - in Schritt (I) mit Heptan, besonders bevorzugt mit n-Heptan oder einem Heptanisomerengemisch mit einem Anteil von mehr als 35 Gew.-% n-Heptan extrahiert.
   Ferner können in Analogie zu EP 1868625 B1 / DE 102005061948 A1 und EP 1868568 B1 folgende ergänzende Verfahrensschritte zur Entfernung von 4'-O-Methylpyridoxin durchgeführt werden:
   (n) Herstellen einer wässrig-ethanolischen Ginkgoextraktlösung mit einem Ethanolgehalt von 40 Gew.-% bis 60 Gew.-% aus der Wasserphase aus Schritt (k) oder aus dem Trockenextrakt aus Schritt (m),
   (o) Auftragen der wässrig-ethanolischen Ginkgoextraktlösung aus Schritt (n) auf einen stark sauren Ionenaustauscher aus Polystyrolharz, an das Sulfonsäuregruppen gebunden sind, um 4'-O-Methylpyridoxin zu entfernen, das an dem lonenaustauscher zurückbleibt und Eluieren unter Verwendung von wässrigem Ethanol, um eine 4'-O-Methylpyridoxin-freie Extraktionslösung als Eluat zu erhalten,
   (p) Konzentrieren des Eluats aus Schritt (o) unter vermindertem Druck und Trocknen bei einer Temperatur von höchstens 60 bis 80°C zu einem Trockenextrakt mit einem Wassergehalt von weniger als 5 Gew.-%.

Beispiele für stark saure Ionenaustauscher sind Merck I und Amberlite IR-120.

Im Folgenden folgen noch einige Definitionen der im Zusammenhang mit dem erfindungsgemäßen Verfahren verwendeten Begriffe:
Trockenextrakt: Trockenextrakte haben gemäß dem Europäischen Arzneibuch im Allgemeinen einen Wassergehalt von höchstens 5 Gew.-%.
Die Bezugnahme auf wasserfrei, zum Beispiel im Zusammenhang mit wasserfreiem Methanol im Schritt (a), bezieht sich im Zusammenhang mit der vorliegenden Erfindung auf einen Wassergehalt von ≤ 1 Gew.-% Wasser.

Extraktion umfasst einstufige, mehrstufige oder kontinuierliche Extraktion.

Bei den aliphatischen Lösungsmitteln mit einem Siedepunkt von 60 bis 100°C handelt es sich bevorzugt um n-Heptan oder um Gemische aus gesättigten acyclischen und/oder cyclischen aliphatischen Kohlenwasserstoffen, die durch ihren Siedepunkt definiert werden und zum Beispiel unter den Bezeichnungen Petrolether oder Petroleumbenzin bekannt und im Handel erhältlich sind. Bevorzugt wird ein aliphatisches Kohlenwasserstoffgemisch verwendet, das im Wesentlichen aus n-Heptan und anderen C7-Alkanen besteht. Ein hoher Anteil an n-Heptan von mehr als 35 Gew.-% n-Heptan (Siedepunkt 98°C) findet sich zum Beispiel in der Fraktion 94-100°C.

Bei dem in Schritt (i) genannten mit Wasser nicht mischbaren Butanol und Pentanol handelt es sich bevorzugt um 1-Butanol oder 1-Pentanol.

Das erfindungsgemäße Verfahren wird nachstehend anhand des Vergleichsbeispiels 1 gemäß EP 431 536 B1 / DE 3940092 A1 und des erfindungsgemäßen Beispiels 1 näher erläutet und beschrieben.

### Ausgangslösung für Beispiel 1 und Vergleichsbeispiel 1

(entsprechend den Verfahrensschritten (a) bis (d) der EP 431 536 B1 und des erfindungsgemäßen Verfahrens)

In den nachfolgenden Beispielen ist unter der Bezeichnung "Heptan" ein gesättigtes aliphatisches Kohlenwasserstoffgemisch zu verstehen mit einem Siedebereich von 94 - 100 °C und einem n-Heptan-Anteil von mehr als 35 Gew.-%.

500 g getrocknete und zerkleinerte Blätter von Ginkgo biloba wurden mit 3.75 kg 60 Gew.-% Aceton 30 min. bei 60 °C extrahiert. Das Pflanzenmaterial wurde abfiltriert, erneut mit 3.75 kg 60 Gew.-% Aceton 30 min. bei 60 °C extrahiert und wiederum abfiltriert. Die beiden so erhaltenen Extraktlösungen wurden vereinigt und eingeengt (438 g; Trockenextraktanteil 36.9 %).

Das resultierende Konzentrat wurde mit 400 g Wasser verdünnt und 1 h bei 12 °C gerührt. Der entstandene Niederschlag wurde abfiltriert und das Filtrat mit 240 g Ammoniumsulfat versetzt und dieses gelöst. Diese Lösung wurde zweimal mit je 400 ml Methylethylketon / Aceton 6/5 (m / m) extrahiert. Die Methylethylketon/Aceton-Phasen wurden vereinigt (808 g).

### Vergleichsbeispiel 1 gemäß EP 431536 B1

(entsprechend den Verfahrensschritten (e) bis (j))

Die Hälfte der obigen Ausgangslösung (404 g) wurde eingeengt (27.2 g; Trockenextraktanteil 50 %). Dieses Konzentrat wurde mit 107.8 g Wasser verdünnt (Trockenextraktanteil 10 %) und dreimal mit je 65 ml wassergesättigtem 1-Butanol ausgeschüttelt. Die 1-Butanol-Phasen wurden vereinigt, eingedampft und im Vakuum 16 h bei 50 °C getrocknet (6.72 g).

Der so erhaltene Trockenextrakt wurde in einer Mischung aus 20.2 g Ethanol und 40.3 g Wasser gelöst (Trockenextraktanteil 10 %). Diese Lösung wurde dreimal mit je 20 ml Heptan ausgeschüttelt, eingedampft und 16 h bei 50 °C im Vakuum getrocknet: 6.11 g (2.4 % bezogen auf die Droge).

| | Gefunden | Ph. Eur. |
|---|---|---|
| Flavonoide | 23.62 % | 22.0 - 27.0 % |
| Bilobalid | 5.48 % | 2.6 - 3.2 % |
| Ginkgolide A, B und C | 5.29 % | 2.8 - 3.4 % |
| Ginkgolsäuren | < 5 ppm | max. 5 ppm |

Der so erhaltene Extrakt entspricht hinsichtlich der Gehalte an Bilobalid und Ginkgoliden A, B und C nicht den Vorgaben des Europäischen Arzneibuchs.

### Erfindungsgemäßes Beispiel 1

### (entsprechend den erfindungsgemäßen Verfahrensschritten (e) bis (m))

Die zweite Hälfte der obigen Ausgangslösung (404 g) wurde eingeengt (20.5 g; Trockenextraktanteil 68.2 %). 10.75 g dieses Konzentrats wurden mit 8.1 g Wasser und 5.6 g Methylethylketon auf einen Trockenextraktanteil von 30 % und einen Methylethylketongehalt von 23 Gew.-% eingestellt und zweimal mit je 18.3 g Methylethylketon / Heptan 8/2 (m / m) ausgeschüttelt.

Die Wasser-Methylethylketon-Phase wurde mit dem verbliebenen Konzentrat der Ausgangslösung (9.75 g) vereinigt. Diese Lösung wurde auf 24.17 g konzentriert, mit 115.5 g Wasser auf einen Trockenextraktanteil von 10 % eingestellt und dreimal mit je 65 ml wassergesättigtem 1-Butanol ausgeschüttelt. Die 1-Butanol-Phasen wurden vereinigt, eingedampft und im Vakuum 16 h bei 50 °C getrocknet (5.93 g).

Der so erhaltene Trockenextrakt wurde in einer Mischung aus 17.8 g Ethanol und 35.6 g Wasser gelöst (Trockenextraktanteil 10 %). Diese Lösung wurde dreimal mit je 20 ml Heptan ausgeschüttelt, eingedampft und 16 h bei 50 °C im Vakuum getrocknet: 5.29 g (2.1 % bezogen auf die Droge).

| | Gefunden | Ph. Eur. |
|---|---|---|
| Flavonoide | 25.95 % | 22.0 - 27.0 % |
| Bilobalid | 3.01 % | 2.6 - 3.2 % |
| Ginkgolide A, B und C | 3.06 % | 2.8 - 3.4 % |
| Ginkgolsäuren | < 5 ppm | max. 5 ppm |

Der so erhaltene Extrakt entspricht hinsichtlich aller Gehalte, insbesondere der Gehalte an Bilobalid und Ginkgoliden A, B und C den Vorgaben des Europäischen Arzneibuchs.

## Patentansprüche

1. Verfahren zur Herstellung eines Trockenextraktes aus Ginkgo biloba-Blättern mit einem Gehalt an 22,0 Gew.-% bis 27,0 Gew.-% Flavonoiden, berechnet als Flavonglykoside 2,6 Gew.-% bis 3,2 Gew.-% Bilobalid, 2,8 Gew.-% bis 3,4 Gew.-% Ginkgolide A, B und C sowie höchstens 5 ppm Ginkgolsäuren, umfassend folgende Schritte:
(a) Extraktion frischer oder getrockneter grüner Blätter von Ginkgo biloba mit wasserhaltigem Aceton, einem wasserhaltigen Alkanol mit 1 bis 3 C-Atomen oder wasserfreiem Methanol bei einer Temperatur von etwa 40 bis 100°C, um eine Rohextraktlösung zu erhalten,
(b) Abtrennen durch Destillation der Hauptmenge des Acetons oder Alkanols mit 1 bis 3 C-Atomen aus der Rohextraktlösung aus Schritt (a) auf einen Gehalt von höchstens 10 Gew.-%, wobei im Fall der Verwendung von wasserfreiem Methanol im Schritt (a), bei den letzten Destillationsstufen Wasser zugesetzt wird, um eine konzentrierte wässrige Lösung zu erhalten,
(c) Verdünnen der konzentrierten wässrigen Lösung aus Schritt (b) mit Wasser auf einen Feststoffgehalt von 5 bis 25 Gew.-%, Abkühlen auf eine Temperatur unter 25°C, Kühlhalten bis zur Bildung eines Niederschlags und Abtrennen des entstandenen Niederschlags, um wiederum eine wässrige Lösung zu erhalten,
(d) Versetzen der erhaltenen wässrigen Lösung aus Schritt (c) mit Ammoniumsulfat und Extraktion der entstandenen Ammoniumsulfat-enthaltenden Lösung mit Methylethylketon oder einem Gemisch aus Methylethylketon und Aceton, Trennung der wässrigen Phase von der Methylethylketon- oder Methylethylketon-Aceton-Phase, um eine Methylethylketon- oder Methylethylketon-Aceton-Phase zu erhalten,
(e) Einengen der Methylethylketon- oder Methylethylketon-Aceton-Phase aus Schritt (d) bis zu einem Trockenextraktanteil von 40 bis 80 Gew.-%, um ein Konzentrat zu erhalten,
(f) Einstellen eines Anteils von 10 bis 60 Gew.-% des Konzentrats aus Schritt (e) mit Wasser und Methylethylketon auf einen Trockenextraktanteil von höchstens 60 Gew.-% und einen Methylethylketongehalt von höchstens 30 Gew.-%, um eine eingestellte Extraktionslösung zu erhalten, und Extraktion der erhaltenen eingestellten Extraktionslösung mit einem Gemisch aus Methylethylketon und einem aliphatischen Lösungsmittel mit einem Siedepunkt von 60 bis 100°C im Verhältnis 7/3 bis 9/1 (m/m), um eine Wasser-Methylethylketon-Phase und eine Methylethylketon-aliphatisches Lösungsmittel-Phase zu erhalten,
(g) Vereinigen des verbliebenen Anteils von 90 bis 40 Gew.-% des Konzentrats aus Schritt (e) mit der Wasser-Methylethylketon-Phase aus Schritt (f) oder der Methylethylketon-aliphatisches Lösungsmittel-Phase aus Schritt (f), um eine Lösung zu erhalten,
(h) Konzentration der Lösung aus Schritt (g) auf einen Feststoffgehalt von 50 bis 70 Gew.-% und Verdünnen des erhaltenen Konzentrats mit Wasser auf einen Feststoffgehalt von höchstens 50 Gew.-%, um eine Lösung zu erhalten,
(i) mehrstufige Extraktion der in Schritt (h) erhaltenen Lösung mit einem mit Wasser nicht mischbaren Butanol oder Pentanol, um eine Butanol- oder Pentanolphase zu erhalten,
(j) Konzentration der Butanol- oder Pentanolphase aus Schritt (i) auf einen Feststoffgehalt von mindestens 50 Gew.-%, um ein Konzentrat zu erhalten,
(k) Verdünnen des in Schritt (j) erhaltenen Konzentrats durch Zugabe derartiger Mengen von Wasser und gegebenenfalls Ethanol, dass eine Lösung von 5 bis 20 Gew.-% Trockenextrakt in Wasser oder höchstens 60 Gew.-% wässrigem Ethanol erhalten wird,
(l) Extraktion der wässrigen oder wässrig-ethanolischen Lösung aus Schritt (k) mit einem aliphatischen Lösungsmittel mit einem Siedepunkt von 60 bis 100°C, Trennung der wässrigen Phase von der aliphatischen Lösungsmittel-Phase, um eine Wasserphase zu erhalten,
(m) Konzentration der in Schritt (I) erhaltenen Wasserphase unter vermindertem Druck und bei einer Temperatur von höchstens 60 bis 80°C, um einen Trockenextrakt mit einem Wassergehalt von weniger als 5 Gew.-% zu erhalten.

2. Verfahren nach Anspruch 1, wobei als Extraktionslösungsmittel in Schritt (a) wasserhaltiges Aceton mit einem Acetongehalt von etwa 50 bis 70 Gew.-% eingesetzt wird.

3. Verfahren nach Anspruch 2, wobei als Extraktionslösungsmittel in Schritt (a) wasserhaltiges Aceton mit einem Acetongehalt von etwa 60 Gew.-% eingesetzt wird.

4. Verfahren nach Anspruch 1, wobei als Extraktionslösungsmittel in Schritt (a) ein wasserhaltiges Alkanol ausgewählt aus Methanol, Ethanol, 1-Propanol und 2-Propanol mit einem Alkanolgehalt von etwa 50 Gew.-% bis 70 Gew.-% eingesetzt wird.

5. Verfahren nach Anspruch 4, wobei als Extraktionslösungsmittel in Schritt (a) wasserhaltiges Ethanol mit einem Ethanolgehalt von etwa 60 Gew.-% eingesetzt wird.

6. Verfahren nach einem der Ansprüche 1 bis 5, wobei in Schritt (c) die verdünnte wässrige Lösung auf eine Temperatur unter 12°C abgekühlt wird.

7. Verfahren nach einem der Ansprüche 1 bis 6, wobei in Schritt (d) mindestens 30 Gew.-% Ammoniumsulfat bezogen auf die wässrige Lösung aus Schritt (c) zugesetzt wird.

8. Verfahren nach einem der Ansprüche 1 bis 7, wobei in Schritt (d) mit einem Gemisch aus Methylethylketon und Aceton im Verhältnis von 7/3 bis 3/4 (m/m) extrahiert wird.

9. Verfahren nach einem der Ansprüche 1 bis 8, wobei in Schritt (i) mit 1-Butanol extrahiert wird.

10. Verfahren nach einem der Ansprüche 1 bis 9, wobei in Schritt (I) mit Heptan extrahiert wird.

11. Verfahren nach Anspruch 10, wobei das Heptan n-Heptan ist oder ein Heptanisomerengemisch mit einem Anteil von mehr als 35 Gew.-% n-Heptan ist.

12. Verfahren nach einem der Ansprüche 1 bis 11, umfassend die folgenden zusätzlichen Schritte:
(n) Herstellen einer wässrig-ethanolischen Ginkgoextraktlösung mit einem Ethanolgehalt von 40 Gew.-% bis 60 Gew.-% aus der Wasserphase aus Schritt (k) oder aus dem Trockenextrakt aus Schritt (m),
(o) Auftragen der wässrig-ethanolischen Ginkgoextraktlösung aus Schritt (n) auf einen stark sauren Ionenaustauscher aus Polystyrolharz, an das Sulfonsäuregruppen gebunden sind, um 4'-O-Methylpyridoxin zu entfernen, das an dem Ionenaustauscher zurückbleibt und Eluieren unter Verwendung von wässrigem Ethanol, um eine 4'-O-Methylpyridoxin-freie Extraktionslösung als Eluat zu erhalten,
(p) Konzentrieren des Eluats aus Schritt (o) unter vermindertem Druck und Trocknen bei einer Temperatur von höchstens 60 bis 80°C zu einem Trockenextrakt mit einem Wassergehalt von weniger als 5 Gew.-%.

## Claims

1. Method for producing a dry extract of Ginkgo biloba leaves having a content of 22.0 % by weight to 27.0 % by weight of flavonoids, calculated as flavon glycosides, 2.6 % by weight to 3.2 % by weight of bilobalide, 2.8 % by weight to 3.4 % by weight of ginkgolides A, B and C and not more than 5 ppm of ginkgolic acids, comprising the following steps:
(a) extraction of fresh or dried green leaves of Ginkgo biloba with acetone containing water, an alkanol with 1 to 3 C atoms containing water or anhydrous methanol at a temperature of about 40 to 100°C to obtain a crude extract solution,
(b) separating by distillation the major amount of the acetone or alkanol with 1 to 3 C atoms from the crude extract solution of step (a) to a content of not more than 10% by weight, wherein, if anhydrous methanol is used in step (a), water is added at the last distillation steps to obtain a concentrated aqueous solution,
(c) diluting the concentrated aqueous solution of step (b) with water to a solid content of 5 to 25 % by weight, cooling to a temperature below 25°C, keeping cool until the formation of a precipitate and separating the formed precipitate to obtain an aqueous solution again,
(d) adding ammonium sulfate to the obtained aqueous solution of step (c) and extraction of the formed solution containing ammonium sulfate with methyl ethyl ketone or a mixture of methyl ethyl ketone and acetone, separating the aqueous phase from the methyl ethyl ketone or methyl ethyl ketone-acetone phase to obtain a methyl ethyl ketone or methyl ethyl ketone-acetone phase,
(e) concentrating the methyl ethyl ketone or methyl ethyl, ketone-acetone phase of step (d) to a dry extract portion of 40 to 80 % by weight to obtain a concentrate,
(f) adjusting a portion of 10 to 60 % by weight of the concentrate of step (e) with water and methyl ethyl ketone to a dry extract portion of not more than 60% by weight and a methyl ethyl ketone content of not more than 30 % by weight to obtain an adjusted extraction solution, and extraction of the obtained adjusted extraction solution with a mixture of methyl ethyl ketone and an aliphatic solvent having a boiling point of 60 to 100°C in a ratio of 7/3 to 9/1 (m/m) to obtain a water-methyl ethyl ketone phase and a methyl ethyl ketone-aliphatic solvent phase,
(g) combining the remaining portion of 90 to 40 % by weight of the concentrate of step (e) with the water-methyl ethyl ketone phase of step (f) or the methyl ethyl ketone-aliphatic solvent phase of step (f) to obtain a solution,
(h) concentrating the solution of step (g) to a solid content of 50 to 70 % by weight and diluting the obtained concentrate with water to a solid content of not more than 50 % by weight to obtain a solution,
(i) multistep extraction of the solution obtained in step (h) with a butanol or pentanol not miscible with water to obtain a butanol or pentanol phase,
(j) concentrating the butanol or pentanol phase of step (i) to a solid content of at least 50 % by weight to obtain a concentrate,
(k) diluting the concentrate obtained in step (j) by the addition of such amounts of water and ethanol, if necessary, that a solution with 5 to 20 % by weight dry extract in water or not more than 60 % by weight aqueous ethanol is obtained,
(l) extraction of the aqueous or aqueous ethanolic solution of step (k) with an aliphatic solvent having a boiling point of 60 to 100°C, separating the aqueous phase from the aliphatic solvent phase to obtain a water phase,
(m) concentrating the water phase obtained in step (1) under reduced pressure and at a temperature of not more than 60 to 80°C to obtain a dry extract with a water content of less than 5% by weight.

2. Method according to claim 1, wherein as extraction solvent in step (a) acetone containing water having an acetone content of about 50 to 70 % by weight is used.

3. Method according to claim 2, wherein as extraction solvent in step (a) acetone containing water having an acetone content of about 60 % by weight is used.

4. Method according to claim 1, wherein as extraction solvent in step (a) alkanol containing water, selected from methanol, ethanol, 1-propanol and 2-propanol, having an alkanol content of about 50 % by weight to 70 % by weight is used.

5. Method according to claim 4, wherein as extraction solvent in step (a) ethanol containing water having an ethanol content of about 60 % by weight is used.

6. Method according to any of claims 1 to 5, wherein in step (c) the diluted aqueous solution is cooled to a temperature below 12°C.

7. Method according to any of claims 1 to 6, wherein in step (d) at least 30 % by weight ammonium sulfate with respect to the aqueous solution of step (c) is added.

8. Method according to any of claims 1 to 7, wherein in step (d) the extraction is effected with a mixture of methyl ethyl ketone and acetone in a ratio of 7/3 to 3/4 (m/m).

9. Method according to any of claims 1 to 8, wherein in step (i) the extraction is effected with 1-butanol.

10. Method according to any of claims 1 to 9, wherein in step (1) the extraction is effected with heptane.

11. Method according to claim 10, wherein the heptane is n-heptane or a mixture of heptane isomers having a portion of more than 35% by weight of n-heptane.

12. Method according to any of claims 1 to 11, comprising the following additional steps:
(n) preparing an aqueous ethanolic Ginkgo extract solution having an ethanol content of 40 % by weight to 60 % by weight from the water phase of step (k) or from the dry extract of step (m),
(o) applying the aqueous ethanolic Ginkgo extract solution of step (n) on a strongly acidic ion exchanger of polystyrene resin to which sulfonic acid groups are bound to remove 4'-O-methyl pyridoxine which remains at the ion exchanger and eluating using aqueous ethanol to obtain an extraction solution free of 4'-O-methyl pyridoxine as eluate,
(p) concentrating the eluate of step (o) under reduced pressure and drying at a temperature of not more than 60 to 80°C to a dry extract having a water content of less than 5% by weight.

## Revendications

1. Procédé de préparation d'un extrait sec des feuilles de ginkgo biloba, contenant:
22,0% à 27,0% en poids de flavonoïdes (calculés sous forme de glycosides de flavone), 2,6% à 3,2% en poids de bilobalide, 2,8% à 3,4% en poids de ginkgolides A, B et C et au maximum 5 ppm d'acides ginkgoliques, comprenant les étapes suivantes:
(a) les feuilles vertes fraîches ou séchées de ginkgo biloba sont extraites à une température d'approximativement 40°C à 100°C avec de l'acétone aqueuse, un alcanol aqueux de 1 à 3 atomes de C ou du méthanol anhydre pour obtenir une solution d'extrait brut;
(b) la majeure partie de l'acétone ou de l'alcanol de 1 à 3 atomes de C est séparée de la solution d'extrait brut de l'étape (a) par distillation à une teneur maximale de 10% en poids, et lorsque du méthanol anhydre est utilisé dans l'étape (a), de l'eau peut être ajoutée au cours des dernières étapes de distillation, pour obtenir une solution aqueuse concentrée;
(c) la solution aqueuse concentrée de l'étape (b) est diluée avec de l'eau à une teneur en matières solides de 5% à 25% en poids, laissée refroidir à une température en dessous de 25°C, maintenue froide jusqu'à ce qu'un précipité se forme, et le précipité résultant est séparé, pour obtenir à nouveau une solution aqueuse concentrée;
(d) du sulfate d'ammonium est ajouté à la solution aqueuse obtenue dans l'étape (c), et la solution résultante contenant du sulfate d'ammonium est extraite avec de la méthyléthylcétone ou un mélange de méthyléthylcétone et d'acétone, la phase aqueuse résultante est séparée de la phase de méthyléthylcétone ou de méthyléthylcétone-acétone pour obtenir une phase de méthyléthylcétone ou de méthyléthylcétone-acétone,
(e) la phase de méthyléthylcétone ou de méthyléthylcétone-acétone de l'étape (d) est concentrée à une teneur en extrait sec de 40% à 80% en poids, pour obtenir un concentré;
(f) une proportion de 10% à 60% en poids du concentré de l'étape (e) est ajusté avec de l'eau et de la méthyléthylcétone à une teneur d'extrait sec maximale de 60% en poids et à une teneur de méthyléthylcétone maximale de 30% en poids pour obtenir une solution d'extraction ajustée, et la solution d'extraction ajustée obtenue est extraite avec un mélange de méthyléthylcétone et d'un solvant aliphatique avec un point d'ébullition de 60°C à 100 C dans un rapport de 7/3 à 9/1 (m/m), pour obtenir une phase d'eau-méthyléthylcétone et une phase de méthyléthylcétone-solvant aliphatique;
(g) la proportion restante de 90% à 40% en poids du concentré de l'étape (e) est combinée avec la phase d'eau - méthyléthylcétone de l'étape (f) ou la phase du méthyléthylcétone - solvant aliphatique de l'étape (f), pour obtenir une solution,
(h) la solution de l'étape (g) est concentrée à une teneur en matières solides de 50% à 70% en poids et le concentré obtenu est dilué avec de l'eau à une teneur en matières solides maximales de 50% en poids, pour obtenir une solution;
(i) la solution obtenue dans l'étape (h) est extraite avec du butanol ou du pentanol étant non-miscible à l'eau dans plusieurs étapes, pour obtenir une phase de butanol ou de pentanol;
(j) la phase de butanol ou de pentanol de l'étape (i) est concentrée à une teneur en matières solides d'au moins 50% en poids pour obtenir un concentré;
(k) le concentré obtenu dans l'étape (j) est dilué avec de l'eau et éventuellement de l'éthanol de manière à obtenir une solution qui contient de 5% à 20% en poids d'extrait sec dans de l'eau ou d'au plus 60% en poids dans de l'éthanol aqueux;
(l) la solution aqueuse ou aqueuse-éthanolique de l'étape (k) est extraite avec un solvant aliphatique avec un point d'ébullition de 60°C à 100°C, la phase aqueuse est séparée de la phase de solvant aliphatique, pour obtenir une phase aqueuse;
(m) la phase aqueuse obtenue dans l'étape (1) est concentrée sous pression réduite et à une température d'au plus 60°C à 80°C, pour obtenir un extrait sec avec une teneur d'eau inférieure à 5% en poids.

2. Procédé selon la revendication 1, dans lequel dans l'étape (a) de l'acétone contenant de l'eau avec une teneur en acétone d'approximativement 50% à 70% en poids est utilisée en tant que solvant d'extraction.

3. Procédé selon la revendication 2, dans lequel dans l'étape (a) de l'acétone contenant de l'eau avec une teneur en acétone d'approximativement 60% en poids est utilisée en tant que solvant d'extraction.

4. Procédé selon la revendication 1, dans lequel dans l'étape (a) un alcanol contenant de l'eau choisi parmi méthanol, éthanol, propane-1-ol et propane-2-ol avec une teneur en alcanol d'approximativement 50% à 70% en poids est utilisé en tant que solvant d'extraction.

5. Procédé selon la revendication 4, dans lequel de l'éthanol contenant de l'eau avec une teneur en éthanol d'approximativement 60% en poids est utilisé en tant que solvant d'extraction dans l'étape (a).

6. Procédé selon l'une quelconque des revendications 1 à 5, dans lequel la solution aqueuse diluée est refroidie à une température en dessous de 12°C dans l'étape (c).

7. Procédé selon l'une quelconque des revendications 1 à 6, dans lequel au moins 30% en poids de sulfate d'ammonium par rapport à la solution aqueuse de l'étape (c) sont ajoutés dans l'étape (d).

8. Procédé selon l'une quelconque des revendications 1 à 7 dans lequel l'extraction dans l'étape (d) est effectuée avec un mélange de méthyléthylcétone et d'acétone dans un rapport de 7/3 à 3/4 (m/m).

9. Procédé selon l'une quelconque des revendications 1 à 8, dans lequel l'extraction dans l'étape (i) est effectuée avec du butane-1-ol.

10. Procédé selon l'une quelconque des revendications 1 à 9, dans lequel l'extraction dans l'étape (1) est effectuée avec du heptane.

11. Procédé selon la revendication 10, dans lequel l'heptane est le n-heptane ou un mélange d'isomères d'heptane avec une proportion supérieure à 35% en poids de n-heptane.

12. Procédé selon l'une quelconque des revendications 1 à 11, comprenant les étapes additionnelles suivantes:
(n) une solution aqueuse-éthanolique d'extrait de ginkgo avec une teneur en éthanol de 40% en poids à 60% en poids est produite à partir de la phase aqueuse de l'étape (k) ou à partir de l'extrait sec de l'étape (m);
(o) la solution d'extrait de ginkgo aqueuse-éthanolique de l'étape (n) est appliquée sur un échangeur d'ions fortement acide constitué de résine de polystyrène, à laquelle des groupes d'acide sulfonique sont liés, pour éliminer du 4'-O-méthylpyridoxine, qui reste à l'échangeur d'ions, et une élution est effectuée en utilisant de l'éthanol aqueux, pour obtenir, en tant que éluat, une solution d'extraction qui est dépourvue de 4'-O-méthylpyridoxine;
(p) les éluats de l'étape (o) sont concentrés sous pression réduite et séchés à une température maximale de 60°C à 80°C à un extrait sec avec une teneur en eau inférieure à 5% en poids.
